# DEMANDE DE BREVET EUROPEEN

(11) **EP 3 639 787 A1**
(43) Date de publication de la demande: **22.04.2020**
(21) Numéro de dépôt: 18200412.7
(22) Date de dépôt: 15.10.2018
(51) Int. Cl.: A61C 13/00, A61C 1/08

(54) **MÉTHODE DE CONCEPTION D'UN ÉLÉMENT PROTHÉTIQUE ET SYSTÈME D'ASSISTANCE D'UN OPÉRATEUR DANS UNE RESTAURATION DENTAIRE**

(71) Demandeur: Dental Design, 1180 Uccle (BE)
(72) Inventeur: CHELALA, Pierre, 1410 Waterloo (BE)
(74) Mandataire: Gevers Patents

(57) **Abrégé**

La présente invention concerne une méthode de conception d'un élément prothétique (1) exécutable préalablement à une taille d'une dent (91) d'un patient pour la pose de l'élément prothétique (1). L'invention concerne aussi un système d'assistance (5) d'un opérateur (D) dans cette taille de la dent (91).

## Description

### Domaine technique

L'invention concerne, entre autres, une méthode de conception d'un élément prothétique ainsi qu'un système d'assistance d'un opérateur dans une restauration dentaire.

### Art antérieur

De nos jours, lorsqu'une restauration dentaire est à effectuer sur une dent d'un patient via la pose d'un élément prothétique, il est usuel pour un dentiste de procéder à une préparation de la dent comprenant, par exemple, une taille de la dent, suivi d'une prise d'empreinte de la dent taillée. Cette empreinte est envoyée dans un laboratoire pour conception de l'élément prothétique, celui-ci étant ensuite essayé, éventuellement adapté, et enfin posé sur la dent taillée du patient.

Malgré l'avènement de méthodes informatiques permettant la prise de cette empreinte numériquement, l'ensemble de ce processus de restauration dentaire est long et nécessite un certain nombre de rendez-vous du patient chez son dentiste.

En outre, la taille de la dent par le dentiste, les matériaux d'empreintes et le flux de fabrication au laboratoire sont des sources régulières d'erreurs techniques mettant en péril l'effet thérapeutique attendu par une restauration dentaire. En particulier, il est complexe pour le dentiste de tenir compte de la structure globale des tissus durs et mous associés à la dentition du patient, de sorte que la taille n'est que rarement parfaitement adaptée à la fois au patient et aux contraintes techniques du prothésiste produisant l'élément prothétique.

### Résumé de l'invention

Un objet de l'invention est de fournir une méthode de conception d'un élément prothétique davantage adapté aux besoins d'un patient, permettant une fabrication et un traitement du patient qui soient plus rapides, moins cher et plus fiables.

À cet effet, l'invention propose une méthode de conception d'un élément prothétique comprenant les étapes suivantes :
(i) fournir un premier fichier informatique comprenant une représentation tridimensionnelle intra-orale d'une dentition comprenant au moins une dent à restaurer au moyen de l'élément prothétique ;
(ii) déterminer une représentation tridimensionnelle d'un extrados de l'élément prothétique sur base du premier fichier informatique ;
(iii) déterminer des paramètres techniques comprenant :
   - un protocole dentaire, et/ou
   - un type de préparation dentaire, et/ou
   - des contraintes techniques,
   sur base du premier fichier informatique ;
(iv) générer un deuxième fichier informatique comprenant une représentation tridimensionnelle d'une réduction volumétrique de la au moins une dent sur base des paramètres techniques ;
(v) valider et/ou modifier le deuxième fichier informatique ;
(vi) obtenir une représentation tridimensionnelle d'un intrados de l'élément prothétique sur base du deuxième fichier informatique validé et/ou modifié ;
(vii) générer un troisième fichier informatique comprenant des informations relatives aux représentations tridimensionnelles des extrados et intrados de l'élément prothétique ;
(viii) produire l'élément prothétique sur base du troisième fichier informatique.

La méthode de conception d'un élément prothétique selon l'invention permet de mettre en oeuvre un processus de restauration dentaire particulièrement efficace, peu coûteux, et rapide, en fournissant un élément prothétique parfaitement adapté aux besoins d'un patient.

En effet, cette méthode concerne principalement des étapes numériques effectuées préalablement à la restauration dentaire. Il est proposé de fournir une (ou plusieurs) représentation tridimensionnelle intra-orale d'une dentition préférentiellement non taillée. Cette étape (i) peut être effectuée en moins de trente secondes au moyen d'un scanner intra-oral connu d'un homme du métier. Les étapes (ii) à (vii) sont au coeur de la présente invention, mais elles sont effectuées au moyen d'outils informatiques, en arrière plan de la restauration dentaire. Elles permettent d'obtenir un modèle numérique d'élément prothétique susceptible d'être parfaitement adapté aux besoins d'un patient sans qu'aucune taille de la au moins une dent soit nécessaire au préalable. L'étape (ii) concerne de façon essentielle l'esthétique externe de l'élément prothétique dont un extrados est déterminé en correspondance avec le premier fichier informatique, et donc de façon globale, avec la dentition du patient et la structure globale des tissus mous et durs associés à cette dentition. Les étapes (iii) à (vii) permettent de déterminer un intrados de l'élément prothétique. Cet intrados doit s'adapter de façon parfaite à une taille de la au moins une dent. Néanmoins, dans le cas de la présente invention, cette taille n'a préférentiellement pas été effectuée, de sorte que la détermination de l'intrados s'effectue essentiellement sur base d'informations du premier fichier informatique, et en particulier sur les paramètres déduits de ces informations, préférentiellement par un informaticien spécialisé et/ou un prothésiste expérimenté. Le deuxième fichier informatique modifié et/ou validé comprenant une représentation tridimensionnelle d'une réduction volumétrique (définissant préférentiellement une taille) de la au moins une dent, ce fichier contient toutes les informations nécessaires à réalisation de cette réduction volumétrique, celle-ci permettant de tailler ultérieurement la au moins une dent de façon à ce que sa surface externe taillée corresponde à l'intrados de l'élément prothétique. Ainsi, et très avantageusement, il est possible de définir numériquement et de produire l'élément prothétique grâce à la méthode de conception selon la présente invention, et, seulement après, de tailler la au moins une dent sur base de la définition de la réduction volumétrique de la au moins une dent contenue dans le deuxième fichier informatique après validation et/ou modification. L'élément prothétique produit est alors plus adapté aux besoins d'un patient, en particulier, à sa dentition et aux tissus mous et durs qui y sont associés, ainsi qu'aux contraintes techniques, par exemple, des tolérances, de fabrication d'un élément prothétique, qu'un autre élément prothétique qui serait produit sur base d'une empreinte de la au moins une dent taillée de façon plus approximative par le dentiste.

Dans la suite proche du résumé, il est commenté précisément sur les étapes de méthode de conception selon l'invention. Il est ensuite présenté différent avantages de cette méthode, notamment dans la mise en oeuvre d'une restauration dentaire.

Les étapes (ii) à (vii) de la méthode de conception selon la présente invention constituent des étapes de planification et préparation intelligentes préalables au bon déroulement d'une restauration dentaire. Différents logiciels connus d'un homme du métier permettent de simuler et/ou de manipuler une image tridimensionnelle d'une dentition restaurée. Il est possible d'obtenir un design d'un extrados probable d'un élément prothétique pour restaurer la au moins une dent à partir d'une telle simulation. Cette obtention de design est néanmoins superficielle car elle n'indique ni comment procéder à une restauration dentaire, ni comment produire un élément prothétique. En outre, de par même son but, ce design ne tient pas compte de nombreux protocoles et tolérances de fabrication d'un élément prothétique adapté aux besoins d'un patient. C'est pourquoi la méthode de conception selon l'invention propose une étude du premier fichier informatique, et donc de la représentation tridimensionnelle intra-orale, pour en déduire les paramètres susmentionnés à l'étape (iii). Ces paramètres comprennent de préférence des informations sur la dentition, les racines dentaires, les gencives, le tissu parodontal, des dents qui sont adjacentes et/ou antagonistes à la au moins une dent, des informations d'alignement et d'encombrement des dents, les zones buccales d'accès, des tolérances de matériaux de conception de l'élément prothétique, ou même des paramètres propres à un centre de production de l'élément prothétique. Toute l'expérience d'un prothésiste est mise au service d'une conception d'un élément prothétique, par l'établissement automatique à l'étape (iv) d'un plan de réduction volumétrique de la au moins une dent prenant en compte l'ensemble de ces paramètres. Cet établissement automatique est préférentiellement réalisé sur base d'un programme d'ordinateur conçu spécialement avantageusement dans le cadre de la présente invention. Cet étape (iv) donne lieu à la génération du deuxième fichier informatique. L'étape (v) de valider et/ou modifier son contenu, et plus spécifiquement la représentation tridimensionnelle de la réduction volumétrique de la au moins une dent. Différents paramètres sont ainsi préférentiellement contrôler par une personne habilitée, par exemple, un prothésiste dentaire.

Ces étapes (iv) et (v) sont très avantageuses car elles permettent de bénéficier d'une aide pour déterminer des paramètres à prendre en compte pour définir virtuellement un élément prothétique tout en permettant une liberté de validation et/ou de modification de ce deuxième fichier informatique obtenu. En particulier, le deuxième fichier informatique est modifiable et n'est pas le résultat d'un logiciel fermé fournissant un design automatique d'élément prothétique non exploitable.

Préférentiellement, le deuxième fichier informatique est un fichier de format STL, plus préférentiellement un fichier CAD modifiable définissant une taille virtuelle de la au moins une dent sur base des étapes (i) à (iii), de sorte qu'il constitue un fichier qui peut être délivré de l'étape (iv), mais également exploité et modifié à l'étape (v) et dans les étapes ultérieures. L'étape (v) comprend préférentiellement une validation et/ou une modification de chacun parmi des paramètres géométriques relatifs à la représentation tridimensionnelle de la réduction volumétrique de la au moins une dent. De préférence, ces paramètres géométriques sont parmi :
- des lignes de marges périphériques,
- des plans parallèles bordant des côtés latéraux de la au moins une dent,
- un contour gingival de la au moins une dent,
- des axes de taille de la au moins une dent,
- des zones de réduction volumétrique de la au moins une dent,
- des faces de réduction volumétrique de la au moins une dent,•
   des grandeurs de réduction volumétrique pour chacune des zone de réduction volumétrique de la au moins une dent.

Tout ces paramètres sont à prendre en compte dans l'établissement d'un élément prothétique. Plus préférentiellement, chacun des paramètres géométriques est modifiable dans un ensemble de valeurs admissibles préalablement défini par au moins un des paramètres techniques déterminé à l'étape (iii). De cette façon, un technicien peu expérimenté ou distrait ne commettra pas d'erreurs dans la conception de l'élément prothétique. Préférentiellement, les zones de réduction volumétrique sont au nombre de trois et divise chaque dent de la gencive, vers son extrémité en trois partie d'environ un tiers de dent. De préférence, une réduction labiale de la au moins une dent est proposée automatiquement à l'étape (iv) dans ces trois zones sous la forme d'une réduction d'une partie cervicale (ou marginale) par un rayon d'épaule compris entre 0,5 mm et 0,8 mm, et une valeur de réduction de mur vertical de -0,2 mm à -2,5 mm, une réduction d'une partie médiane par cette valeur, et une réduction d'une partie occlusale (ou incisale) par cette valeur, avec un axe d'inclinaison compris entre 1° et 45°. De préférence, une réduction proximale de la au moins une dent est comprise entre -0,4 mm et -1,5 mm. De préférence, les faces de réductions volumétriques sont parmi une face occlusale, une face linguale, une face mésiale et une face distale. Chaque face peut préférentiellement être réduite selon un tiers marginal, un tiers moyen et un tiers incisai, divisant la face en trois tiers le long d'une hauteur dirigée d'une racine et/ou d'un contour gingival vers une extrémité incisive de la au moins une dent.

Le deuxième fichier informatique ainsi validé et/ou modifié comprend par conséquent une représentation tridimensionnelle de la réduction volumétrique de la au moins une dent, et donc peut servir de base à l'obtention en l'étape (vi) d'une représentation tridimensionnelle de l'intrados de l'élément prothétique, celui-ci étant complémentaire à une surface externe de la au moins une dent réduite de façon volumétrique. Le deuxième fichier informatique et l'ensemble des représentations ainsi définies de l'intrados et de l'extrados de l'élément prothétique constituent préférentiellement des fichiers informatiques CAD connus d'un homme du métier mais avantageusement exploitable selon l'invention. Le design de l'élément prothétique étant défini, il est alors généré un troisième fichier informatique, de préférence de type CAM, comprenant des informations relatives à ces représentations tridimensionnelles d'intrados et d'extrados définissant le design de l'élément prothétique. Ces informations servent de base à la production de l'élément prothétique à l'étape (viii) sans perdre la possibilité d'exploiter les informations du deuxième fichier informatique pour guider un praticien dans la taille de la au moins une dent afin de correspondre avec la réduction volumétrique précédemment définie.

Ce troisième fichier informatique se distingue du deuxième fichier informatique en ce qu'il comprend des informations techniques préférentiellement directement exploitables lors d'une production de l'élément prothétique, notamment par une machine de production, alors que le deuxième fichier informatique comprend préférentiellement des données de représentation tridimensionnelles. L'obtention du troisième fichier informatique à partir du deuxième ne se fait pas par des logiciels existants mais à partir d'un programme d'ordinateur selon l'invention pour tenir compte à nouveau des paramètres techniques, et préférentiellement très spécifiquement pour tenir compte de protocoles de conception de l'éléments prothétique et de protocoles dentaires d'usinage dentaire. De préférence, le troisième fichier informatique est également un fichier de stéréolithographie.

De façon très préférée, les informations du troisième fichier informatique comprennent des instructions d'usinage d'un matériau, et l'étape (viii) comprend une sous-étape d'usinage du matériau sur base des instructions d'usinage. De cette façon, une machine d'usinage apte à lire les instructions peut usiner un matériau pour lui donner une forme creuse comprenant une face interne analogue à la représentation de l'intrados et une forme externe analogue à la représentation de l'extrados. Le matériau comprend préférentiellement au moins un parmi de la céramique, du zircone, de la céramique hybride, du composite, de la résine, et ce de façon à concevoir un élément prothétique solide et biocompatible, permettant au patient de bénéficier d'une restauration dentaire saine et stable. Préférentiellement, le matériau consiste en de la biocéramique adaptée à un usinage.

De préférence, l'étape (ii) de la méthode comprend les sous-étapes suivantes :
(ii.1) choisir une représentation tridimensionnelle modèle d'une dentition modèle dans une base de données sur base du premier fichier informatique ;
(ii.2) choisir une zone de la représentation tridimensionnelle modèle correspondant à une zone de la représentation tridimensionnelle intra-orale correspondant à la au moins une dent ;
(ii.3) valider et/ou modifier la zone de la représentation tridimensionnelle modèle sur base du premier fichier informatique ;
(ii.4) définir la représentation tridimensionnelle de l'extrados de l'élément prothétique à partir de la zone validée et/ou modifiée de la représentation tridimensionnelle modèle.

Cette réalisation préférée de la méthode comprend de façon essentielle un choix de représentation tridimensionnelle d'une dentition sans défaut simulant la représentation tridimensionnelle de la dentition comprenant la au moins une dent parmi une base de donnée (ou bibliothèque) de telles représentations. Ainsi, il est possible de déterminer une forme finale de dentition restaurée adaptée aux besoins d'un patient, et d'en tirer la forme d'une enveloppe externe à donner à l'élément prothétique pour optimiser l'esthétique de cette nouvelle forme finale de dentition. Cette enveloppe externe consiste de façon basique en l'extrados de l'élément prothétique. Vu qu'il se peut qu'aucune représentation tridimensionnelle modèle ne corresponde exactement à l'extrados souhaité, il est prévu une étape de validation et/ou modification analogue à celle de l'étape (v) détaillée ci-dessus pour permettre à un prothésiste dentaire et/ou un CAD designer d'effectuer des retouches sur cette zone. Celles-ci peuvent par exemple concerner un axe d'insertion, un axe et/ou une hauteur de contact de la au moins une dent avec des dents antagonistes et/ou adjacentes, ou encore un alignement dentaire. Il est préférentiellement tenu compte d'un index gingival pour définir la représentation tridimensionnelle de l'extrados. Cet index gingival est de préférence compris dans les contraintes techniques déterminées à l'étape (iii).

De préférence, la méthode de conception selon l'invention est telle que le premier fichier informatique comprend également une image radiographique de la dentition, l'étape (i) comprend une sous-étape de comparaison de la représentation tridimensionnelle intra-orale avec l'image radiographique.

Avantageusement, l'étape (i) permet de fournir davantage d'informations sur la dentition et les tissus mous et durs associés à un prothésiste dentaire et/ou un CAD designer pour définir plus précisément les besoins du patient, et donc un élément prothétique davantage adapté par les étapes (ii) à (vii). L'image radiographique est de préférence une image bidimensionnelle. Une image radiographique tridimensionnelle (par exemple, une image produite par une technique d'imagerie volumétrique par faisceau conique, ou CBCT) n'est cependant pas exclus du cadre de l'invention. Avantageusement, l'image radiographique permet à un prothésiste dentaire d'identifier la chambre pulpaire, et le système nerveux associé à la au moins une dent du patient, et de définir des paramètres permettant une réduction volumétrique adaptée de la au moins une dent. Cette sous-étape est particulièrement avantageuse dans le cadre d'une planification intelligente d'une taille de la au moins une dent. Cette sous-étape de comparaison se réalise de façon pratique de préférence via une superposition de l'image radiographique et de la représentation tridimensionnelle intra-orale, et un repérage d'axes. En outre, une telle image radiographique est utile dans le cas d'une restauration dentaire nécessitant une retaille d'une certaine dent, par exemple, dans le cas d'un retrait d'un ancien élément prothétique. Ainsi, il est plus aisé d'identifier la certaine dent, ainsi que les corps mous et durs qui lui sont associés. Plus préférentiellement et précisément, au moins un des paramètres techniques est déterminé à l'étape (iii) sur base de la comparaison de cette sous-étape de l'étape (i).

Préférentiellement, selon ce mode de réalisation préféré, l'étape (i) comprend une identification d'axes de référence communs sur la représentation tridimensionnelle intra-orale et sur l'image radiographique, et la comparaison comprend une superposition de ces axes de référence pour figer les repères pour un travail numérique adéquat sur la au moins une dent aux étapes (ii) à (vii).

De façon générale, la méthode de conception selon l'invention permet une inversion d'une étape globale de préparation de la au moins une dent avec une conception de l'élément prothétique, et ce dans un processus global de restauration dentaire qui se distingue alors très avantageusement de l'art antérieur, en ce qu'il est considérablement plus efficace et rapide. En particulier, ce processus de restauration dentaire propose une préparation de la au moins une dent comprenant une taille de la au moins une dent adaptée à l'élément prothétique et non l'inverse ! Un exemple d'un mode de réalisation très préféré d'un tel processus de restauration dentaire à partir de la méthode de conception selon l'invention est décrit dans la description détaillée ci-après. Plus spécifiquement, la longue séquence de rendez-vous entre le patient et le dentiste peut de façon avantageuse être remplacée par un rendez-vous unique au cours duquel, d'une part, la au moins une dent est taillée par un dentiste en fonction d'informations du deuxième fichier informatique, et, d'autre part, l'élément prothétique produit est posé sur la au moins une dent taillée. Par exemple, dans le cas où l'élément prothétique est une couronne d'une dent, cette restauration dentaire peut être effectuée lors d'un seul rendez-vous d'une durée inférieure à trente minutes. La taille de la au moins une dent sur base des informations du deuxième fichier informatique est davantage exacte et précise, car elle est basée sur des informations rigoureuses, étudiées et analysées par un ou plusieurs spécialistes, et ce sur base de méthode numériques fiables, loin des conditions stressantes de travail du dentiste lors d'une opération de chirurgie dentaire. La comptabilité entre la taille de la au moins une dent, et l'élément prothétique, d'une part, et la structure globale des tissus et de la dentition, d'autre part, est ainsi assurée et parfaitement prédictible. Aucune prise d'empreinte ne doit être effectuée après la taille de la au moins une dent car il est acquis que l'élément prothétique sera adapté à cette taille avec une grande précision, à la condition où cette préparation est effectuée sur base d'informations du deuxième fichier informatique.

Avantageusement, la méthode de conception selon l'invention permet donc d'éviter de nombreux aller-retour de l'élément prothétique entre un cabinet dentaire et un laboratoire où il est conçu, de tels aller-retour étant parfois nécessaire selon l'état de la technique pour des adaptations de l'élément prothétique, lorsqu'un essai de pose de celui-ci sur la dent du patient n'est pas concluant. En outre, et avantageusement, la méthode de conception selon l'invention permet une large diminution de souffrances d'un patient devant subir une restauration dentaire vu que l'élément prothétique peut être placé parfaitement en une seule séance.

Avantageusement, la méthode de conception selon l'invention permet de produire des éléments prothétiques possédant les avantages susmentionnés à bas coût pour le patient car des étapes d'empreintes dentaires et/ou de modifications éventuelles de l'élément prothétique produit sur base d'une taille de la au moins une dent ne sont plus effectuées. Il est estimé une réduction avantageuse de ce coût à un tiers d'un montant de conception du même élément prothétique au moyen de techniques selon l'art antérieur. Pour une couronne, ce coût est estimé à 300 €. Le coût global d'une restauration dentaire imputable à la production d'un élément prothétique est donc très fortement réduit, rendant ainsi les restaurations dentaires davantage démocratiques pour une clientèle défavorisée.

La méthode de conception selon l'invention, et le processus de restauration dentaire sous-jacent répondent avantageusement à un besoin sociétal grandissant compte tenu du vieillissement accéléré de certaines populations, notamment en Europe, aux USA, au Canada ou en Asie. La méthode de conception selon l'invention permet de produire rapidement et efficacement des éléments prothétiques d'une grande qualité et parfaitement adaptés aux besoins de chaque patient, répondant ainsi à une demande croissante de restauration dentaire tout en compensant pleinement une pénurie dans le personnel soignant, notamment prothésistes ou chirurgiens-dentistes.

Avantageusement, la méthode de conception selon l'invention permet de limiter l'impact écologique et environnemental dû à une délocalisation d'une production d'éléments prothétiques. En effet, il est possible d'exécuter la méthode de conception selon l'invention de façon locale, au sein d'un laboratoire ou d'un cabinet dentaire, pour produire des éléments prothétiques à bas coût, sans avoir recours à une main d'oeuvre étrangère et bon marché. En outre, la méthode de conception étant de façon principale numérique, il peut être joint une étape de vérification d'un respect des normes de sécurité et de traçabilité propres à un élément prothétique.

Une réalisation particulière du processus susmentionné peut être établie sur base d'un quatrième fichier informatique tel que décrit ci-dessous. En effet, selon un mode de réalisation préféré de la méthode de conception, cette méthode comprend en outre l'étape supplémentaire :
(vii') générer un quatrième fichier informatique sur base du deuxième fichier informatique validé et/ou modifié,
   le quatrième fichier informatique comprenant des instructions d'usinage de la au moins une dent correspondant à la représentation tridimensionnelle de la réduction volumétrique de la au moins une dent.

Ce quatrième fichier informatique est préférentiellement aussi un fichier CAM, de préférence de stéréolithographie, généré sur base du deuxième fichier informatique de la même façon que ce que le troisième fichier informatique est généré à l'étape (vii), mais en tenant pleinement compte de protocole dentaire pour une taille de la au moins une dent.

Le quatrième fichier informatique a un grand avantage sur le deuxième fichier informatique de comprendre directement des instructions d'usinage de la au moins une dent utilisable par une machine d'usinage appropriée et/ou un praticien. Il est ainsi plus aisé de suivre les instructions d'usinage que de définir soi-même un usinage à partir d'un fichier CAD de représentation de réduction volumétrique de la au moins une dent. Ce quatrième fichier informatique constitue un élément informatique majeur de l'invention car il propose pour la première fois un usinage exacte de la au moins une dent permettant d'obtenir une surface externe usinée de la au moins une dent correspondant exactement à l'intrados de l'élément prothétique. Il est ainsi possible d'éviter tout espace vide entre la au moins une dent taillée et l'élément prothétique. La présence d'un tel espace vide serait néfaste à l'intégrité dentaire du patient car elle permettrait une prolifération de bactéries.

Dans le cadre du présent document, un « élément prothétique » fait préférentiellement référence à un élément parmi : une couronne, un pont, une obturation, un inlay, un onlay, un implant dentaire, une facette, ou une combinaison de plusieurs de ces éléments. Le terme d'élément prothétique englobe tout type de prothèses fixes, et tout type de prothèses amovibles partiellement ou complètement sur au moins une dent et/ou sur au moins un implant dentaire. Bien qu'une partie du présent document soit tout particulièrement illustré sur base d'une restauration dentaire comprenant une conception et/ou une pose d'une couronne, une restauration dentaire au moyen d'un autre élément prothétique ne saurait se départir du cadre de l'invention. Dans le cadre du présent document, les termes de « intrados » et « extrados » d'un élément prothétique font référence respectivement à une surface intérieure et une surface extérieure de l'élément prothétique, et sont connus d'un homme du métier prothésiste dentaire et/ou dentiste.

Dans le cadre de ce document, les termes de « dentiste », « chirurgien-dentiste », ou plus généralement de « praticien » sont de préférence utilisés de façon interchangeables. Le praticien responsable d'une capture d'une représentation tridimensionnelle intra-oral et/ou d'une image radiographique est préférentiellement un dentiste. Le praticien responsable d'une taille de la au moins une dent est préférentiellement un dentiste ou chirurgien-dentiste. Un praticien responsable de la réalisation des étapes (ii) à (vii) est préférentiellement un prothésiste dentaire, spécialisé dans la conception numérique d'éléments prothétiques.

Dans le cadre de ce document, le format STL est un format de fichier informatique connu d'un homme du métier. L'abréviation STL correspond à « Standard Triangle Language » en anglais.

L'usage, dans le présent document, du verbe « comprendre » de ses variantes, ainsi que ses conjugaisons, ne peut en aucune façon exclure la présence d'éléments autres que ceux mentionnés. L'usage, dans le présent document, de l'article indéfini « un », « une », ou de l'article défini « le », « la » ou « l' », pour introduire un élément n'exclut pas la présence d'une pluralité de ces éléments.

L'invention propose également un élément prothétique produit par la méthode de conception selon l'invention. L'ensemble des modes de réalisation préférés ainsi que l'ensemble des avantages de la méthode de conception selon l'invention se transposent mutatis mutandis au présent élément prothétique. En particulier, l'élément prothétique est de préférence constitué d'un matériau de biocéramique pour assurer une compatibilité biologique avec les tissus mous et durs, et la dentition d'un patient, ainsi qu'une très grande longévité de l'élément prothétique.

L'invention propose également des outils pour mettre en oeuvre la méthode de conception selon l'invention : un ensemble d'appareils, des programmes d'ordinateurs et des supports lisibles par un ordinateur. Ces outils sont détaillés ci-dessous.

La présente invention propose :
- un premier programme d'ordinateur comprenant des premières instructions qui, lorsque le premier programme d'ordinateur est exécuté, conduisent à mettre en oeuvre l'étape (iv) de la méthode de conception selon l'invention ;
- un deuxième programme d'ordinateur comprenant des deuxièmes instructions qui, lorsque le deuxième programme d'ordinateur est exécuté, conduisent à mettre en oeuvre l'étape (vii) de la méthode de conception selon l'invention ;
- un troisième programme d'ordinateur comprenant des troisièmes instructions qui, lorsque le troisième programme d'ordinateur est exécuté, conduisent à mettre en oeuvre l'étape supplémentaire (vii') de la méthode de conception selon un mode de réalisation très préféré de l'invention.

L'invention propose également un ensemble de programmes d'ordinateur comprenant le premier et/ou le deuxième et/ou le troisième programmes d'ordinateur. L'invention propose également un support lisible par un ordinateur sur lequel est enregistré au moins un programme d'ordinateur présent dans un ensemble de programmes d'ordinateur selon l'invention.

Tel que détaillé précédemment, les premier, deuxième et troisième programmes d'ordinateur sont au coeur de la méthode de conception selon l'invention. Ils permettent d'automatiser partiellement la conception d'un élément prothétique, d'assister numériquement un prothésiste dans la définition de l'élément prothétique, et de planifier intelligemment sa production. En particulier, l'ensemble des modes de réalisation ainsi que l'ensemble des avantages de la méthode de conception ayant trait aux étapes (iv), (vii) et (vii') selon l'invention se transposent mutatis mutandis aux présents programmes et ensembles de programme d'ordinateur, ainsi qu'au support lisible par un ordinateur.

La présente invention propose un ensemble d'appareils pour concevoir un élément prothétique par exécution de la méthode de conception selon l'invention, l'ensemble d'appareils comprenant :
- au moins un appareil d'imagerie pour mettre en oeuvre l'étape (i) de la méthode de conception ;
- un système informatique comprenant :
   - une interface pour recevoir :
      les paramètres techniques déterminés à l'étape (iii) de la méthode de conception, et
      des validations et/ou des modifications du deuxième fichier
      informatique de l'étape (v) de la méthode de conception ;
      et pour visualiser et/ou communiquer des données sur les représentations tridimensionnelles :
      intra-orale du premier fichier informatique fourni à l'étape (i) ;
      de l'extrados de l'élément prothétique obtenue à l'étape (ii) ;
      de la réduction volumétrique de la au moins une dent du deuxième fichier informatique généré à l'étape (iv) ;
      de la réduction volumétrique de la au moins une dent du deuxième fichier informatique validé et/ou modifié à l'étape (v) ;
      de l'intrados de l'élément prothétique obtenue à l'étape (vi) ;
   - une unité logique pour mettre en oeuvre au moins partiellement les étapes (ii), (iv), (vi) et (vii) de la méthode de conception ;
- une machine de production pour lire les informations du troisième fichier informatique généré à l'étape (vii), et pour mettre en oeuvre l'étape (viii) de la méthode de conception.

Il est clair que divers avantages et modes de réalisation préférés de l'ensemble d'appareils selon l'invention se déduisent directement de l'ensemble des modes de réalisation préférés ainsi que l'ensemble des avantages de la méthode de conception selon l'invention. En particulier, le au moins un appareil d'imagerie comprend de préférence au moins un parmi : un scanner intra-oral et/ou un appareil de radiographie par rayons X. Préférentiellement, les informations du troisième fichier informatique consiste en des instructions d'usinage d'un matériau et la machine de production comprend une machine d'usinage pour lire le troisième fichier informatique et exécuter ces instructions. Une telle machine d'usinage est connue d'un homme du métier et la fourniture d'instructions dans un fichier informatique compatible est l'objectif de l'étape (vii). Préférentiellement, la machine d'usinage est apte à concevoir plusieurs éléments prothétiques simultanément. Préférentiellement, le système informatique comprend un ordinateur dont un écran, un clavier et une souris sont l'interface et dont le processeur et au moins un support lisible sur lequel est enregistré au moins un programme d'ordinateur parmi les premier, deuxième et/ou troisième programmes d'ordinateur, définissent l'unité logique.

La présente invention propose un support lisible par ordinateur sur lequel est enregistré un deuxième fichier informatique généré à l'étape (iv) et/ou validé et/ou modifié à l'étape (v) de la méthode de conception selon l'invention. La présente invention propose également un support lisible par ordinateur sur lequel est enregistré au moins un parmi un troisième fichier informatique étant généré à l'étape (vii) de la méthode de conception selon l'invention, et/ou un quatrième fichier informatique étant généré à l'étape (vii') du mode de réalisation préféré de la méthode de conception selon l'invention.

Les avantages et modes de réalisation préférés de ces étapes (iv), (v), (vii) et (vii'), ainsi que des deuxième, troisième et quatrième fichiers informatiques se transposent mutatis mutandis aux présents supports lisibles par ordinateur. De préférence, le support lisible par ordinateur sur lequel est enregistré le troisième fichier informatique permet un transfert physique du troisième fichier informatique d'un centre informatique de données comprenant un système informatique tel que susmentionné pour l'exécution des étapes (ii) à (vii), et (vii') de la méthode de conception, vers un laboratoire comprenant une machine de production pour lire ce troisième fichier informatique et produire l'élément prothétique. De préférence, le support lisible par ordinateur sur lequel est enregistré le quatrième fichier informatique permet un transfert physique du quatrième fichier informatique du centre informatique de données, vers un cabinet dentaire ou un centre médical où un dentiste pourra le lire par ordinateur afin de s'informer des instructions d'usinage de la au moins une dent à exécuter.

Il est introduit ci-dessous une autre partie de la présente invention consistant en un système d'assistance d'un opérateur dans un placement d'un élément prothétique préférentiellement conçu par la méthode de conception. Cette autre partie pourrait fait l'objet d'une invention en tant que telle, et ce indépendamment des différents aspects techniques et de la méthode de conception introduits précédemment.

En particulier, un autre objet de l'invention est de fournir un système d'assistance d'un opérateur pour effectuer une taille d'au moins une dent d'une dentition d'un patient sans erreurs techniques.

À cet effet, l'invention propose un système d'assistance d'un opérateur dans une restauration dentaire comprenant :
- un robot muni d'un bras robotisé mobile et solidaire d'un outil d'usinage placé en une extrémité du bras robotisé ;
- un système de guidage spatial du robot comprenant :
   - un premier repère spatial fixé au bras robotisé ;
   - un deuxième repère spatial configuré pour être attaché en un point d'une zone d'opération ;
   - un détecteur configuré pour déterminer une première distance séparant le détecteur du premier repère spatial, et une deuxième distance séparant le détecteur du deuxième repère spatial ;
   - une unité informatique logique configurée pour :
      lire un fichier informatique comprenant des instructions d'usinage d'au moins une dent,
      recevoir des données concernant les première et deuxième distances, et
      déterminer des informations de comparaison des données avec les instructions d'usinage ;
- un outil de communication connecté numériquement à l'unité informatique pour communiquer les informations de comparaison à l'opérateur.

Le système d'assistance selon l'invention permet à un opérateur, de préférence un dentiste et/ou chirurgien-dentiste, d'effectuer une taille de la moins une dent sans erreurs techniques.

En effet, l'outil de communication permet de communiquer à l'opérateur des informations de comparaison déterminées par l'unité informatique logique entre d'une part, des instructions d'usinage de la au moins une dent d'un fichier informatique, et d'autre part, une position relative entre le bras robotisé du robot et une zone d'opération, via des repères spatiaux dont il est évalué une distance les séparant par l'intermédiaire du détecteur. De cette façon, il peut être rendu compte d'écarts entre les instructions d'usinage prédéterminées et l'action réelle d'usinage via une position du bras robotisé. L'opérateur obtient ainsi un compte rendu de ses actions au moyen du robot de façon à suivre exactement les instructions d'usinage et à obtenir une taille précise et sans erreurs techniques.

Le système de guidage est une composante essentielle du système d'assistance car sans lui, il est impossible d'associer une action du robot et une instruction d'usinage en tenant pleinement compte de variation possible de position du patient et d'un repère attaché à la zone d'opération. Préférentiellement, le système de guidage permet d'informer en temps réel l'opérateur et/ou le robot d'un mouvement compensatoire à effectuer par rapport à un mouvement d'un patient, par exemple, dû à sa respiration, à des mouvements du maxillaire ou de la mandibule.

De préférence, de façon générale, par zone d'opération, il faut entendre une zone peu étendue d'une bouche d'un patient comprenant au moins une dent à restaurer.

Préférentiellement, le robot est connecté numériquement à l'unité informatique logique, de sorte que cette dernière est apte à contrôler une exécution des instructions d'usinage au moyen de l'outil d'usinage. Ainsi, et très avantageusement, le robot peut procéder automatiquement à la taille de la au moins une dent suivant les instructions d'usinage. Le robot est néanmoins préférentiellement semi-automatique dans le sens où il guide l'opérateur dans l'exécution de la taille de la au moins une dent. Ainsi, il assiste l'opérateur, par exemple, en le guidant par un mouvement et/ou une rotation et/ou une vibration du bras robotisé, ou encore en arrêtant automatiquement l'outil d'usinage en cas de sortie de la zone d'opération nuisible à l'intégrité physique d'un patient.

Avantageusement, le robot permet une précision inégalable seule par l'opérateur de l'ordre de 20 microns, ainsi qu'une réduction d'un temps d'intervention pour la taille de la au moins une dent de l'ordre de 40 à 60%. Avantageusement, le système d'assistance permet donc à l'opérateur d'effectuer une taille de la moins une dent avec une grande précision et sans fatigue mentale ou physique. Dans le cadre de ce document, la au moins une dent comprend optionnellement de façon générale au moins dix dents, et/ou au moins vingt-quatre dents d'un patient. Le système d'assistance permet avantageusement à l'opérateur d'effectuer la restauration d'un tel grand nombre de dents, en une séance, sans fatigue.

Préférentiellement, l'opérateur interagit avec le bras robotisé, en le sens que le bras robotisé est également apte à être guidé dans une certaine mesure par l'opérateur, par exemple pour être amené en zone d'opération. L'opérateur dispose également préférentiellement d'un action d'activation et de désactivation de l'outil d'usinage et/ou du robot. De façon combiné avec le mode de réalisation préféré précédent, le bras robotisé peut ainsi être amené dans la zone d'opération par l'opérateur, puis travailler de façon semi-autonome sous un contrôle d'exécution des instructions d'usinage par l'unité informatique logique. Avantageusement, cette double interaction du bras robotisé avec l'opérateur permet une grande flexibilité, une grande sécurité et une grande précision, pour l'opérateur dans une opération de restauration dentaire.

Préférentiellement, l'outil d'usinage est une fraise dentaire et le terme d'usinage fait référence à un fraisage.

Préférentiellement, le robot comprend six roulements pour subir des rotations partielles autour de six axes. Il comprend des roues pour se mouvoir sur un sol d'un cabinet dentaire.

Selon un mode de réalisation préféré de l'invention, le fichier informatique consiste en un quatrième fichier informatique généré à l'étape (vii') selon le mode de réalisation très préféré de la méthode de conception selon l'invention.

En particulier, dans ce cas, le système d'assistance selon l'invention permet à l'opérateur de tenir compte de la dentition globale et des tissus durs et mous d'un patient, la taille de la au moins une dent étant ainsi parfaitement adaptée aux besoins du patient et à un élément prothétique précédemment conçu par exécution de la méthode de conception selon l'invention. Le système d'assistance selon l'invention permet donc de mettre en oeuvre un processus de restauration dentaire précédemment défini dans ce résumé et duquel il est transposé les avantages au présent système d'assistance, le fichier informatique est un tel quatrième fichier informatique.

Selon un mode de réalisation préféré du système d'assistance, l'outil de communication comprend un écran pour afficher en temps réel, au moins partiellement, la zone d'opération et l'outil d'usinage. De préférence, les informations de comparaison comprennent en outre une position et/ou une orientation de l'outil d'usinage dans la zone d'opération.

Un tel outil de communication permet de guider l'opérateur dans une préparation dentaire en vue d'une restauration dentaire, par exemple, en guidant virtuellement une manipulation à effectuer sur l'écran ou en proposant plusieurs positions et/ou orientation de l'outil d'usinage dans la zone d'opération, ou même plusieurs trajectoires possibles de l'outil d'usinage dans la zone d'opération déterminée virtuellement via l'unité informatique logique sur base du fichier informatique.

De façon optionnelle, le deuxième repère spatial comprend au moins un parmi :
- une partie supérieure comprenant un casque et/ou au moins une sangle apte à être disposé autour d'un front d'un patient ;
- une partie inférieure comprenant une mentonnière apte à enfermer un menton d'un patient ;
et des deuxièmes symboles cibles détectables par le détecteur fixés sur la partie supérieure et/ou la partie inférieure.

Plus optionnellement, le deuxième repère spatial comprend les parties inférieure et supérieure. Avantageusement, le deuxième repère spatial permet de repérer individuellement une mâchoire supérieure et une mâchoire inférieure d'un patient. En effet, la partie supérieure suit les mouvements de la mâchoire supérieure couplée au front, alors que la partie inférieure suit les mouvements de la mâchoire inférieure couplée au menton. En outre, ce deuxième repère spatial peut de façon avantageuse optionnellement être utilisé pour une classe de patient.

Optionnellement, selon une nouvelle réalisation du deuxième repère spatial, celui-ci comprend un écarteur de joues d'un patient rigide apte à forcer une rigidification d'une bouche d'un patient, permettant un accès à la zone d'opération, comprenant des deuxièmes symboles cibles sur une portion supérieure et sur une portion inférieure, ces symboles étant apte à être détecté par le détecteur. Cette nouvelle réalisation optionnelle permet également un repérage des mâchoires inférieure et supérieure d'un patient via le détecteur.

Selon un mode de réalisation préféré du système d'assistance, le premier repère spatial comprend des premiers symboles cibles détectables par le détecteur ; et le deuxième repère spatial comprend :
- une partie dentaire apte à épouser au moins partiellement une forme d'une portion d'une dentition comprise dans la zone d'opération ;
- un élément protubérant comprenant des deuxièmes symboles cibles détectables par le détecteur, l'élément protubérant étant configuré pour être attaché à la partie dentaire au moyen d'un système d'attache, de façon à s'étendre hors de la zone d'opération.

Avantageusement, un tel deuxième repère spatial permet un usage d'une partie dentale propre à la dentition de chaque patient, alors que l'élément protubérant peut être réutilisé d'un patient à un autre et attacher au moyen du système d'attache sur une partie dentaire. Un autre avantage de la partie dentaire est qu'elle épouse au moins partiellement une forme de la portion de la dentition dans la zone d'opération, de sorte que l'ensemble du deuxième repère spatial est davantage stable par rapport au repère du patient, ce qui est primordiale dans l'objectif du système de guidage spatial du robot. Un deuxième repère spatial d'un autre type, par exemple, basé sur des symboles cibles qui seraient collés sur des dents de la dentition ne se départirait pas du cadre de l'invention. Néanmoins, la caractéristique protubérante de l'élément protubérant est avantageuse car cet élément s'étend hors de la zone d'opération, ce qui facilite la détection des deuxièmes symboles cibles par le détecteur.

Selon une réalisation particulière, la partie dentaire est d'une forme hémicylindrique. La partie dentaire constitue alors une gouttière sensiblement courbe apte à être disposée sur la dentition.

Préférentiellement, la partie dentaire comprend une empreinte dentaire partielle conçu à partir d'une représentation tridimensionnelle intra-orale de la dentition, plus préférentiellement au moyen d'un scanner intra-oral. Plus préférentiellement, selon le mode de réalisation préféré pour lequel le fichier informatique est un quatrième fichier informatique obtenu selon une étape (vii') de la méthode de conception, la représentation tridimensionnelle intra-orale est celle fournie à l'étape (i). Compte tenu de l'usage temporaire de la partie dentaire, celle-ci peut-être réalisé en un matériau moins résistant que l'élément prothétique, et par exemple, au moyen de techniques d'usinage ou de processus de fabrication additive tel qu'une impression tridimensionnelle.

### Brève description des figures

D'autres caractéristiques et avantages de la présente invention apparaîtront à la lecture de la description détaillée qui suit pour la compréhension de laquelle on se reportera aux figures annexées parmi lesquelles :
- la figure 1 illustre une vue schématique un organigramme d'une méthode de conception d'un élément prothétique selon un mode de réalisation préféré de l'invention ;
- la figure 2 illustre une vue schématique d'une mise en oeuvre d'une méthode de restauration dentaire comprenant une méthode de conception d'un élément prothétique selon un mode de réalisation préféré de l'invention ;
- la figure 3 illustre une vue en perspective simplifiée d'une salle de dentisterie équipée avec un système d'assistance d'un opérateur dans une restauration dentaire selon un mode de réalisation préféré de l'invention ;
- la figure 4 illustre une vue tridimensionnelle d'éléments d'un robot et d'un système de guidage spatial du robot selon un mode de réalisation préféré de l'invention.

Les dessins des figures ne sont pas à l'échelle. Généralement, des éléments semblables sont dénotés par des références semblables dans les figures. Dans le cadre du présent document, les éléments identiques ou analogues peuvent porter les mêmes références. En outre, la présence de numéros ou lettres de référence aux dessins ne peut être considérée comme limitative, y compris lorsque ces numéros ou lettres sont indiqués dans les revendications.

### Description détaillée de modes de réalisation particuliers de l'invention

La présente invention est décrite avec des réalisations particulières et des références à des figures mais l'invention n'est pas limitée par celles-ci. Les dessins ou figures décrits ne sont que schématiques et ne sont pas limitants.

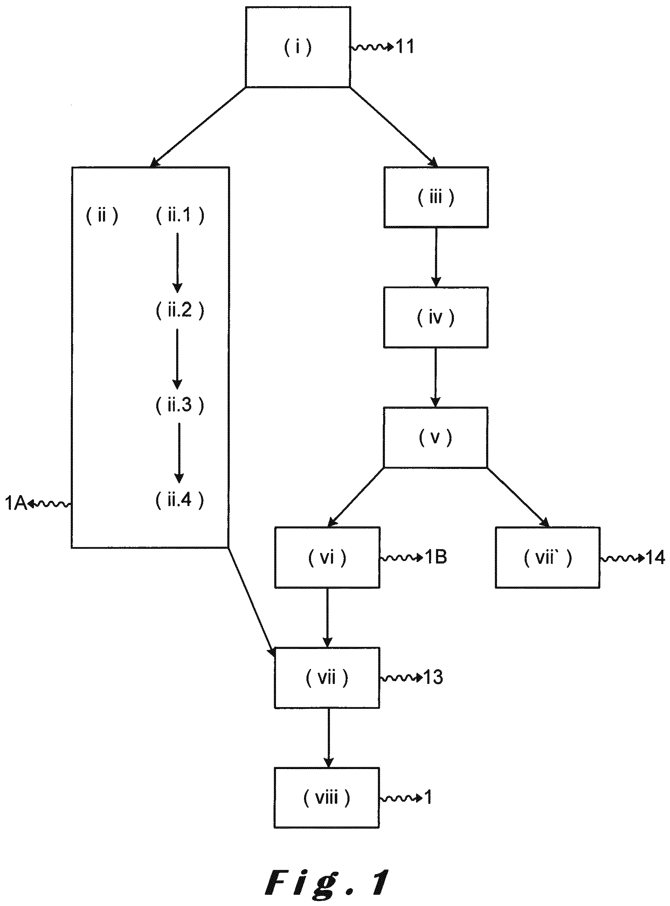

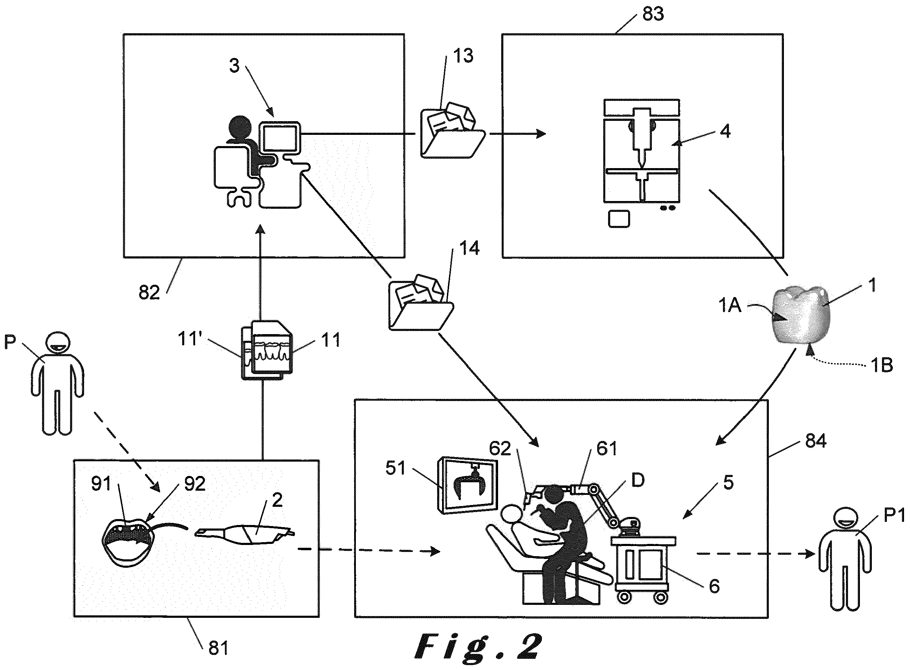

Les figures 1 et 2 illustrent au moins partiellement une mise en oeuvre d'une méthode de restauration dentaire d'une dent 91 d'une dentition 92 d'un patient P comprenant une méthode de conception d'un élément prothétique 1 selon un mode de réalisation préféré de l'invention. Il est ci-après fait référence aux notations (i), (ii), (iii), ... des différentes étapes de la méthode de conception introduites dans le résumé de l'invention ainsi que dans les revendications.

Il est supposé que le patient P doit subir une restauration dentaire comprenant un fraisage de la dent 91 et la pose d'un élément prothétique consistant en une couronne 1 sur la dent 91 fraisée. La méthode de conception selon l'invention est appliquée à la couronne 1 à concevoir. Cette méthode permet, selon un mode de réalisation préféré, d'être complété de façon à pouvoir restaurer facilement et rapidement la dent 91 du patient P.

Pour cela, la méthode propose au patient P de se rendre dans un centre d'imagerie tridimensionnelle 81 et de subir un scan intra-oral de sa dentition 92 au moyen d'un scanner intra-oral 2, et ce de façon à exécuter l'étape (i), c'est-à-dire, fournir un premier fichier informatique 11 comprenant une représentation tridimensionnelle intra-orale de la dentition 92 comprenant la dent 91 à restaurer au moyen de la couronne 1. Le centre d'imagerie tridimensionnelle 81 est, de façon préférée, un parmi : un cabinet dentaire, un centre médical, ou encore un centre d'imagerie de proximité. Avantageusement, un tel scan prend moins de cinq minutes pour être effectué ; après ce laps de temps, la patient P est libéré. Il est préférentiellement complété par une image radiographique panoramique bidimensionnelle de la bouche et/ou de la dentition 92 du patient P, et qui est soit jointe au premier fichier informatique 11, soit fournie simultanément au premier fichier informatique 11 dans un autre premier fichier informatique 11'. L'image radiographique panoramique est de préférence capturée au moyen d'un appareil à rayons X. Elle permet de fournir davantage d'information sur la structure de la dentition 92 du patient P, sur les nerfs, les tissus mous et durs associés, et ce notamment par une comparaison de la représentation tridimensionnelle intra-orale avec l'image radiographique.

Le premier fichier informatique 11 est ensuite envoyé, via un support lisible par ordinateur et/ou via un nuage de données partagées, dans un centre de données 82 comprenant un système informatique 3 permettant une exécution des étapes (ii) à (vii), et (vii') de la méthode de conception selon le mode de réalisation préféré de l'invention, dans l'ordre illustré en figure 1, et tel que détaillé dans le résumé de l'invention. Un prothésiste dentaire et/ou un designer CAD spécialisé traitent le premier fichier informatique 11 pour déterminer une représentation de la couronne 1, celle-ci comprenant des représentations tridimensionnelles 1A et 1B respectivement de son extrados et de son intrados, obtenues d'après les étapes (ii) et (vi) respectivement. Cette représentation est comprise dans un ou plusieurs fichiers informatiques de format STL de type CAD conçus au moins partiellement au moyen d'un premier programme d'ordinateur selon la présente invention. Un deuxième et un troisième programmes d'ordinateur selon un mode de réalisation préféré de l'invention permet d'utiliser des protocoles dentaires et de prothèse pour générer un troisième 13 et un quatrième 14 fichiers informatiques de type CAM qui comprennent des instructions d'usinage respectivement d'un matériau en céramique pour former la couronne 1, et de la dent 91 pour s'adapter à l'intrados 1B de la couronne 1 lors du placement.

Le troisième fichier informatique 13 est ensuite envoyé, via un support lisible par ordinateur et/ou via un nuage de données partagées, dans un laboratoire 83 comprenant une machine d'usinage 4 permettant une exécution de l'étape (viii) de la méthode de conception selon le mode de réalisation préféré de l'invention. En particulier, la machine d'usinage 4 lit le troisième fichier informatique 13 et exécute les instructions d'usinage de façon à usiner le matériau en céramique pour former la couronne 1, l'extrados et l'intrados de la couronne 1 consistant en des surfaces d'une forme correspondant aux représentations tridimensionnelles 1A et 1B.

Le quatrième fichier informatique 14 est, quant à lui, envoyé, via un support lisible par ordinateur et/ou un nuage de données partagées, dans un cabinet dentaire 84 d'un praticien chirurgien et/ou dentiste D équipé d'un système d'assistance 5 selon un mode de réalisation de l'invention. Il est à noter qu'un praticien D possédant une telle machine d'usinage 4 dans le cabinet dentaire 84 peut recevoir le troisième fichier informatique 13 et produire lui-même la couronne 1 dont il a besoin. En particulier, les lieux 81, 83 et 84 sont susceptibles de coïncider.

Le praticien D, disposant du quatrième fichier informatique 14, réalise alors l'opération de restauration dentaire en fraisant la dent 91 et en fixant la couronne 1 sur la dent fraisée. Cette opération est bien connue, mais elle est sensiblement améliorée dans le cadre de l'invention car la dent 91 est fraisée avec l'aide du système d'assistance 5 et de façon à s'adapter à la couronne 1 déjà conçue, et non l'inverse. Ainsi, l'opération est d'une durée avantageuse de moins de trente minute et le patient P' retrouve un sourire à la sortie du cabinet dentaire.

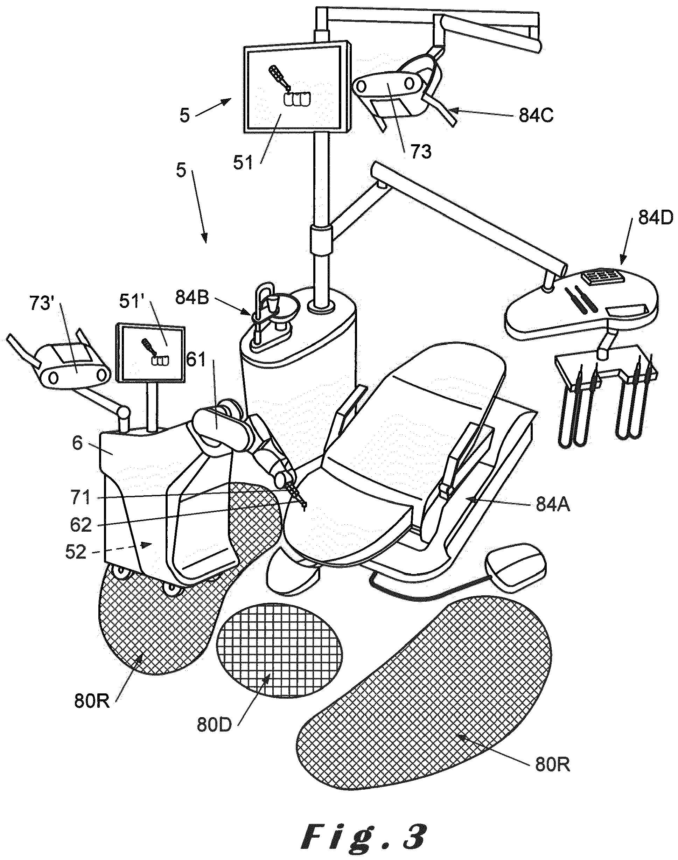

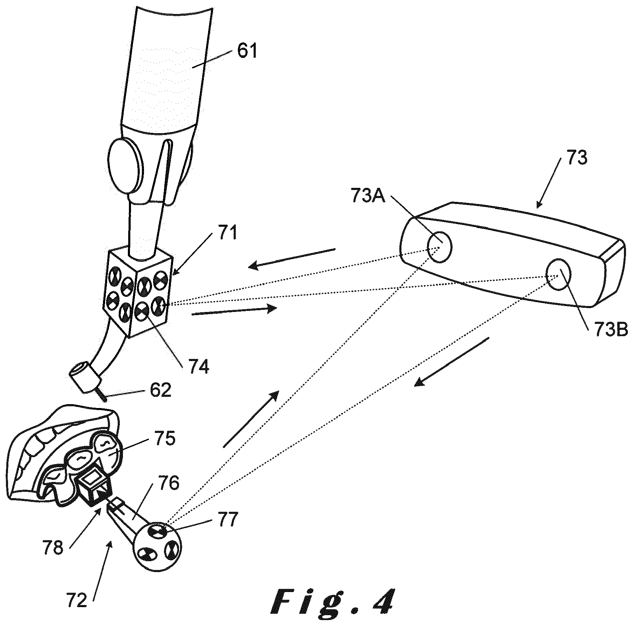

Le procédé menant à la restauration dentaire est maintenant décrit au regard de la figure 3 illustrant une vue simplifiée d'une salle d'opération du cabinet dentaire 84 équipée d'un système d'assistance 5 selon un mode de réalisation préféré de l'invention. Les éléments du cabinet dentaire 84 représentés en figure 2 sont tous illustrés en figure 3. La salle comprend des outils techniques classiques du praticien D tels qu'un siège 84A pour y accueillir le patient P, une station 84B comprenant une alimentation d'eau et une tour portant une lampe 84C placée en hauteur, et un bras mobile s'étendant autour du siège 84A, et auquel est couplé mécaniquement une palette 84D d'instruments standards du praticien D. Le système d'assistance 5 comprend un robot 6 mobile autour du siège 84A, dans une zone 80R. Il est muni de roues pour assurer cette mobilité. Il est également muni d'un bras articulé robotisé 61 mobile et solidaire d'un outil d'usinage consistant en une fraise 62 placée en une extrémité du bras robotisé 61. Le système d'assistance 5 comprend également un système de guidage spatial du robot 6 comprenant un détecteur 73, préférentiellement fixé à un support de la lampe 84C, en surplomb du siège 84A. Tel qu'illustré en figure 4, le détecteur 73 est configuré pour déterminer :
- une première distance séparant un de ses points d'au moins un parmi des premiers symboles cibles 74 d'un premier repère spatial 71 fixé au bras robotisé 61 ;
- une deuxième distance séparant le un de ses points d'au moins un parmi des deuxièmes symboles cibles 77 d'un deuxième repère spatial 72 qui est attaché en un point d'une zone d'opération de la dentition 92.

Pour permettre au détecteur 73 de déterminer ces première et deuxième distances avec profondeur, dans une direction quelconque de l'espace, il est prévu deux caméras 73A et 73B espacées sur le détecteur 73. Le deuxième repère spatial 72 comprend une partie dentaire 75 apte à épouser une forme d'une portion de la dentition 92 comprise dans la zone d'opération, cette portion ne comprenant pas la dent 91, et un élément protubérant 76 comprenant les deuxièmes symboles cibles 77. La partie dentaire 75 et l'élément protubérant sont pourvus d'un système d'attache 78 femme-mâle permettant d'emboîter par clips une extrémité mâle de l'élément protubérant 76 dans une extrémité femelle de la partie dentaire 75, et ce pour réutiliser avantageusement l'élément protubérant 76 d'une opération dentaire à l'autre, tout en jetant la partie dentaire 75 après une utilisation. De préférence, la partie dentaire est conçu d'un matériau solide et bon marché à partir du première fichier informatique 11 et ce pour constituer une empreinte dentaire partielle s'adaptant parfaitement à la portion de la dentition 92. La partie dentaire 75 est, par exemple, conçue par un processus de fabrication additive. Il est nécessaire de concevoir l'élément protubérant comme s'étendant hors de la zone d'opération pour facilité la visibilité des deuxièmes symboles cibles 77 par le détecteur 73 tout en garantissant que le deuxième repère spatial 72 rend compte des mouvement du patient P relativement à un repère fixe de la salle et au bras robotisé 61. Le système de guidage spatial comprend enfin une unité informatique logique 52 comprenant un processeur agencé au sein d'une carcasse du robot 6, de façon à limiter l'encombrement du système d'assistance 5. L'unité informatique logique 52 est configurée pour :
- lire le quatrième fichier informatique 14,
- recevoir des données concernant les première et deuxième distances du détecteur 73, et
- déterminer des informations de comparaison des données avec les instructions d'usinage du quatrième fichier informatique 14,
- contrôler et/ou arrêter le robot 6 et/ou la fraise 62 en fonction des informations de comparaison.

Le système d'assistance 5 comprend également un outil de communication 51 connecté numériquement à l'unité informatique 52 et comprenant un écran pour communiquer les informations de comparaison au praticien D. Ainsi, et tel que détaillé dans le résumé de l'invention, le praticien D peut être guidé dans son fraisage par le système d'assistance 5 de façon à garantir une grande fiabilité et une grande rapidité de l'opération, ainsi qu'une grande précision du fraisage, et en particulier, une obtention d'une surface de la dent 91 fraisée correspondant exactement à l'intrados de la couronne 1 à placer. Optionnellement, le robot 6 comprend un autre bras mécanique mobile comprenant un autre détecteur 73' d'une structure et pour un usage semblables au détecteur 73. Optionnellement, le robot 6 comprend un autre outil 51' d'une structure et d'un usage semblables à l'outil de communication 51. En particulier, il est connecté numériquement à l'unité informatique 52 pour communiquer et/ou recevoir les informations de comparaison au praticien D. Il consiste de préférence en un parmi un ordinateur portable, ou une tablette interactive, pour le praticien D. Durant l'opération de chirurgie dentaire, le praticien D agissant en tant opérateur assisté est essentiellement situé dans une zone 80D, entourée de façon latérale par la zone 80R.

En résumé, l'invention concerne, une méthode de conception d'un élément prothétique 1 exécutable préalablement à une taille d'une dent 91 d'un patient pour la pose de l'élément prothétique 1. L'invention concerne aussi un système d'assistance 5 d'un opérateur D dans cette taille de la dent 91.

La présente invention a été décrite en relation avec des modes de réalisations spécifiques, qui ont une valeur purement illustrative et ne doivent pas être considérés comme limitatifs. D'une manière générale, il apparaîtra évident pour un homme du métier que la présente invention n'est pas limités aux exemples illustrés et/ou décrits ci-dessus. L'invention comprend chacune des caractéristiques nouvelles ainsi que toutes leurs combinaisons.

## Revendications

1. Méthode de conception d'un élément prothétique (1) comprenant les étapes suivantes :
(i) fournir un premier fichier informatique (11) comprenant une représentation tridimensionnelle intra-orale d'une dentition (92) comprenant au moins une dent (91) à restaurer au moyen dudit élément prothétique (1) ;
(ii) déterminer une représentation tridimensionnelle d'un extrados (1A) dudit élément prothétique (1) sur base dudit premier fichier informatique (11) ;
(iii) déterminer des paramètres techniques comprenant :
- un protocole dentaire, et/ou
- un type de préparation dentaire, et/ou
- des contraintes techniques,
sur base dudit premier fichier informatique (11) ;
(iv) générer un deuxième fichier informatique comprenant une représentation tridimensionnelle d'une réduction volumétrique de ladite au moins une dent (91) sur base desdits paramètres techniques ;
(v) valider et/ou modifier ledit deuxième fichier informatique ;
(vi) obtenir une représentation tridimensionnelle d'un intrados (1B) dudit élément prothétique (1) sur base dudit deuxième fichier informatique validé et/ou modifié ;
(vii) générer un troisième fichier informatique (13) comprenant des informations relatives aux représentations tridimensionnelles desdits extrados (1A) et intrados (1B) dudit élément prothétique (1) ;
(viii) produire ledit élément prothétique (1) sur base dudit troisième fichier informatique (13).

2. Méthode de conception selon la revendication précédente **caractérisée en ce que** :
- ledit deuxième fichier informatique consiste en un fichier de format STL modifiable ;
- l'étape (v) comprend une validation et/ou une modification de chacun parmi des paramètres géométriques relatifs à ladite représentation tridimensionnelle de la réduction volumétrique de ladite au moins une dent (91), lesdits paramètres géométriques étant parmi :
• des lignes de marges périphériques,
• des plans parallèles bordant des côtés latéraux de ladite au moins une dent (91),
• un contour gingival de ladite au moins une dent (91),
• des axes de taille de ladite au moins une dent (91),
• des zones de réduction volumétrique de ladite au moins une dent (91),
• des faces de réduction volumétrique de ladite au moins une dent (91),
• des grandeurs de réduction volumétrique pour chacune desdites zone de réduction volumétrique de ladite au moins une dent (91),
chacun desdits paramètres géométriques étant modifiable dans un ensemble de valeurs admissibles préalablement défini par au moins un desdits paramètres techniques.

3. Méthode de conception selon l'une quelconque des revendications précédentes **caractérisée en ce que** :
- lesdites informations dudit troisième fichier informatique (13) comprennent des instructions d'usinage d'un matériau ;
- l'étape (viii) comprend une sous-étape d'usinage dudit matériau sur base desdites instructions d'usinage.

4. Méthode de conception selon l'une quelconque des revendications précédentes **caractérisée en ce que** :
- ledit premier fichier informatique (11) comprend également une image radiographique de ladite dentition (92) ;
- l'étape (i) comprend une sous-étape de comparaison de ladite représentation tridimensionnelle intra-orale avec ladite image radiographique ;
- au moins un desdits paramètres techniques est déterminé à l'étape (iii) sur base de la comparaison de cette sous-étape.

5. Méthode de conception selon l'une quelconque des revendications précédentes, comprenant en outre l'étape supplémentaire :
(vii') générer un quatrième fichier informatique (14) sur base dudit deuxième fichier informatique validé et/ou modifié, ledit quatrième fichier informatique (14) comprenant des instructions d'usinage de ladite au moins une dent (91) correspondant à ladite représentation tridimensionnelle de ladite réduction volumétrique de ladite au moins une dent (91).

6. Ensemble d'appareils pour concevoir un élément prothétique (1) par exécution de la méthode de conception selon l'une quelconque des revendications précédentes, l'ensemble d'appareils comprenant :
- au moins un appareil d'imagerie (2) pour mettre en oeuvre l'étape (i) de la méthode de conception ;
- un système informatique (3) comprenant :
• une interface pour recevoir :
les paramètres techniques déterminés à l'étape (iii) de la méthode de conception, et
des validations et/ou des modifications du deuxième fichier informatique de l'étape (v) de la méthode de
conception ;
et pour visualiser et/ou communiquer des données sur :
la représentation tridimensionnelle intra-orale du premier fichier informatique (11) fourni à l'étape (i) ;
la représentation tridimensionnelle de l'extrados (1A) dudit élément prothétique (1) obtenue à l'étape (ii) ;
la représentation tridimensionnelle de la réduction volumétrique de la au moins une dent (91) du deuxième fichier informatique généré à l'étape (iv) ;
la représentation tridimensionnelle de la réduction volumétrique de la au moins une dent (91) du deuxième fichier informatique validé et/ou modifié à l'étape (v) ;
la représentation tridimensionnelle de l'intrados (1B) dudit élément prothétique (1) obtenue à l'étape (vi) ;
• une unité logique pour mettre en oeuvre au moins partiellement les étapes (ii), (iv), (vi) et (vii) de la méthode de conception ;
- une machine de production (4) pour lire les informations du troisième fichier informatique (13) généré à l'étape (vii), et pour mettre en oeuvre l'étape (viii) de la méthode de conception.

7. Ensemble de programmes d'ordinateur comprenant :
- un premier programme d'ordinateur comprenant des premières instructions qui, lorsque ledit premier programme d'ordinateur est exécuté, conduisent à mettre en oeuvre l'étape (iv) de la méthode de conception selon l'une quelconque des revendications 1 à 5.
- un deuxième programme d'ordinateur comprenant des deuxièmes instructions qui, lorsque ledit deuxième programme d'ordinateur est exécuté, conduisent à mettre en oeuvre l'étape (vii) de la méthode de conception selon l'une quelconque des revendications 1 à 5.

8. Ensemble de programmes d'ordinateur selon la revendication précédente, comprenant en outre un troisième programme d'ordinateur comprenant des troisièmes instructions qui, lorsque ledit troisième programme d'ordinateur est exécuté, conduisent à mettre en oeuvre l'étape supplémentaire de la méthode de conception selon la revendication 5.

9. Support lisible par un ordinateur sur lequel est enregistré au moins un programme d'ordinateur présent dans un ensemble de programmes d'ordinateur selon l'une quelconque des revendications 7 à 8.

10. Support lisible par un ordinateur sur lequel est enregistré au moins un parmi :
un troisième fichier informatique (13) généré par la méthode de conception selon l'une quelconque des revendication 1 à 5, et un quatrième fichier informatique (14) généré par la méthode de conception selon la revendication 5.

11. Elément prothétique (1) produit par la méthode de conception selon l'une quelconque des revendication 1 à 5.

12. Système d'assistance (5) d'un opérateur (D) dans une restauration dentaire comprenant :
- un robot (6) muni d'un bras robotisé (61) mobile et solidaire d'un outil d'usinage (62) placé en une extrémité dudit bras robotisé (61) ;
- un système de guidage spatial dudit robot comprenant :
• un premier repère spatial (71) fixé audit bras robotisé (62) ;
• un deuxième repère spatial (72) configuré pour être attaché en un point d'une zone d'opération ;
• un détecteur (73) configuré pour déterminer une première distance séparant ledit détecteur (73) dudit premier repère spatial (71), et une deuxième distance séparant ledit détecteur (73) dudit deuxième repère spatial (72) ;
• une unité informatique logique (52) configurée pour :
lire un fichier informatique comprenant des instructions d'usinage d'au moins une dent (91),
recevoir des données concernant lesdites première et deuxième distances, et
déterminer des informations de comparaison desdites données avec lesdites instructions d'usinage ;
- un outil de communication (51) connecté numériquement à ladite unité informatique pour communiquer lesdites informations de comparaison audit opérateur (D).

13. Système (5) selon la revendication précédente **caractérisé en ce que** ledit fichier informatique consiste en un quatrième fichier informatique (14) généré par la méthode de conception selon la revendication 5.

14. Système (5) selon l'une quelconque des revendications 12 à 13 **caractérisé en ce que** ledit robot (6) est connecté numériquement à ladite unité informatique logique, de sorte que cette dernière soit apte à contrôler une exécution desdites instructions d'usinage au moyen dudit outil d'usinage (62).

15. Système selon l'une quelconque des revendications 12 à 14 **caractérisé en ce que** ledit premier repère spatial (71) comprend des premiers symboles cibles (74) détectables par ledit détecteur (73) ; et **en ce que** ledit deuxième repère spatial (72) comprend :
- une partie dentaire (75) apte à épouser au moins partiellement une forme d'une portion d'une dentition (92) comprise dans ladite zone d'opération ;
- un élément protubérant (76) comprenant des deuxièmes symboles cibles (77) détectables par ledit détecteur (73), ledit élément protubérant (76) étant configuré pour être attaché à ladite partie dentaire (75) au moyen d'un système d'attache (78), de façon à s'étendre hors de ladite zone d'opération.
